# EUROPÄISCHE PATENTSCHRIFT

(11) **EP 1 070 723 B1**
(45) Veröffentlichungstag und Bekanntmachung des Hinweises auf die Patenterteilung: **25.02.2004**
(21) Anmeldenummer: 00114362.7
(22) Anmeldetag: 05.07.2000
(51) Int. Cl.: C07F 9/06, C07F 9/59, C07F 9/572, C07F 9/6533, C07F 9/6509, C07B 39/00

(54) **Aminophosphoniumverbindungen**
Aminophosphonium compounds
Composés aminophosphonium

(30) Priorität: 23.07.1999 DE 19934595
(43) Veröffentlichungstag der Anmeldung: 24.01.2001
(73) Patentinhaber: Clariant GmbH, 65929 Frankfurt am Main (DE)
(72) Erfinder: Schiemenz, Berthold, Dr., 65929 Frankfurt (DE); Wessel, Thomas, Dr., 60386 Frankfurt (DE); Pfirmann, Ralf, Dr., 64347 Griesheim (DE)

(56) Entgegenhaltungen:
- WO-A-98/22413
- WO-A-98/32532
- WO-A-99/11588
- ZAL'TSMAN I.S.: "Reaction of (1-aziridinyl) phosphonium chlorides with amines" JOURNAL OF GENERAL CHEMISTRY USSR., Bd. 57, Nr. 10, 20. März 1988 (1988-03-20), Seiten 2032-2036, XP002149812 CONSULTANTS BUREAU. NEW YORK., US

## Beschreibung

Die vorliegende Erfindung betrifft neue Aminophosphoniumverbindungen, diese enthaltende Stoffgemische, ihre Herstellung und Verwendung.

Aminophosphoniumverbindungen finden, wie aus WO 98/32532 und WO 98/22413 hervorgeht, bei Herstellung von Fluor enthaltenden Verbindungen mittels einer Halogen-Fluor-Austauschreaktion (Halex-Reaktion) als Katalysatoren Anwendung. Das in WO 98/32532 und WO 98/22413 verwendete Tetrakis-(diethylamino)phosphoniumbromid liefert zwar gute Ergebnisse, weist aber eine sehr hohe dermale Toxizität auf. Die sehr hohe dermale Toxizität steht einer technischen Anwendung allerdings entgegen.

Es besteht die Aufgabe, neue Verbindungen bereitzustellen, die sich als Katalysator oder Bestandteil von Katalysatorsystemen für Phasentransferreaktionen, insbesondere für Halogen-Fluor-Austauschreaktionen eignen, eine geringere dermale Toxizität besitzen und die die unter Verwendung von Tetrakis(diethylamino)-phosphoniumbromid erzielbaren Ergebnisse erreichen oder sogar noch übertreffen.

Gelöst wird diese Aufgabe durch Verbindungen der allgemeinen Formel 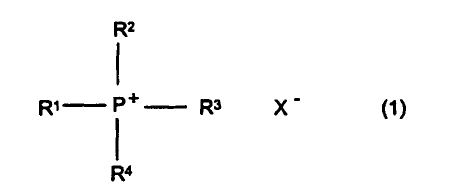 worin einer, zwei oder drei der Reste R¹, R², R³ und R⁴ für 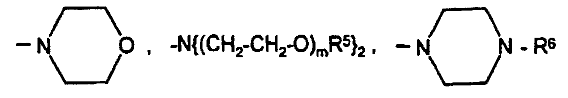 oder -N{(CH₂-CH₂-N(R⁷))ₙR⁸}₂ stehen, wobei m und n eine ganze Zahl von 1 bis 10 bedeuten, R⁵, R⁶, R⁷ und R⁸ unabhängig voneinander gleich oder verschieden sind und einen geradkettigen oder verzweigten Alkylrest mit 1 bis 10 C-Atomen bedeuten, und der oder die verbleibenden Reste R¹ bis R⁴ für 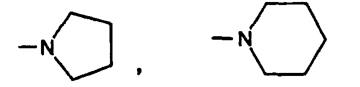 oder -NR⁹R¹⁰, wobei R⁹ und R¹⁰ gleich oder verschieden sind und einen geradkettigen oder verzweigten Alkylrest mit 1 bis 10 C-Atomen bedeuten, stehen und X⁻ ein anorganisches oder organisches Anion oder ein Äquivalent eines mehrwertigen anorganischen oder organischen Anions ist.

Von Interesse sind Verbindungen der Formel (1), worin ein oder zwei der Reste R¹, R², R³ und R⁴ für 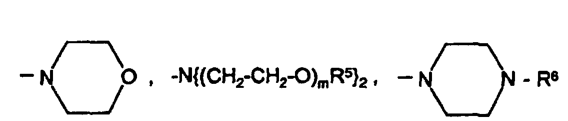 oder -N{(CH₂-CH₂-N(R⁷))ₙR⁸}₂ und die verbleibenden Reste R¹ bis R⁴ für 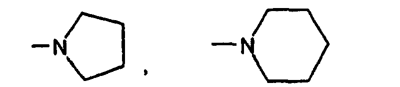 oder -NR⁹R¹⁰ stehen.

Von Bedeutung sind femer Verbindungen der Formel (1), worin m und n eine ganze Zahl von 1 bis 6 bedeuten und R⁵, R⁶, R⁷, R⁸, R⁹ und R¹⁰ gleich oder verschieden sind und für einen geradkettigen oder verzweigten Alkylrest mit 1 bis 4 C-Atomen stehen.

Die vorstehend erwähnten Reste R¹, R², R³ und R⁴ stehen insbesondere für 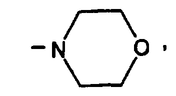 -N{(CH₂-CH₂-O)ₘR⁵}₂ oder -N{(CH₂-CH₂-N(R⁷))ₙR⁸}₂ bevorzugt für -N{(CH₂-CH₂-O)ₘR⁵}₂ oder -N{(CH₂-CH₂-N(R⁷))ₙR⁸}₂, besonders bevorzugt für -N{(CH₂-CH₂-O)ₘR⁵}₂.

Der oder die vorstehend genannten verbleibenden Reste R¹ bis R⁴ stehen insbesondere für

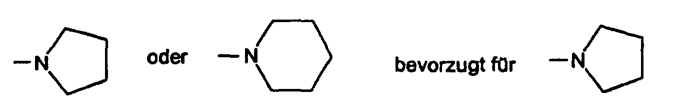

In den Verbindungen der Formel (1) steht X⁻ für F⁻, Cl⁻, Br⁻, J⁻, ClO₄ ⁻, BF₄⁻, PF₆⁻, NO₃⁻, HSO₄⁻, ½ SO₄²⁻, H₂PO₄⁻, ½ HPO₄²⁻, 1/3 PO₄³⁻, R'-COO⁻, wobei R' einen Alkylrest mit 1 bis 9 C-Atomen, einen Phenylrest, Benzylrest oder Naphthylrest bedeutet, R''-SO₃⁻, wobei R" einen Alkylrest mit 1 bis 18 C-Atomen, einen Phenylrest, Tolylrest oder Naphthylrest bedeutet, HCO₃⁻, ½ CO₃²⁻ oder für ½ C₆H₄(COO⁻)₂.

X⁻ bedeutet insbesondere F⁻, Cl⁻, Br⁻, J⁻, BF₄⁻, PF₆⁻ oder ½ SO₄ ²⁻, bevorzugt F⁻, Cl⁻, oder Br⁻, besonders bevorzugt Cl⁻.

Folgende Aminophosphoniumverbindungen (Aminophosphoniumsalze) seien als Beispiele genannt, ohne Anspruch auf Vollständigkeit zu erheben.
Bis(2-methoxyethyl)amino-tris(pyrrolidino)phosphoniumchlorid
Di(bis(2-methoxyethyl)amino)-bis(pyrrolidino)phosphoniumchlorid
Tri(bis(2-methoxyethyl)amino)-(pyrrolidino)phosphoniumchlorid
Bis(methyl-diethoxyethylen)amino-tris(pyrrolidino)phosphoniumchlorid
Di(bis(methyl-diethoxyethylen)amino)-bis(pyrrolidino)phosphoniumchlorid
Tri(bis(methyl-diethoxyethylen)amino)-(pyrrolidino)phosphoniumchlorid
Bis(methyl-tetraethoxyethylen)amino-tris(pyrrolidino)phosphoniumchlorid
Di(bis(methyl-tetraethoxyethylen)amino)-bis(pyrrolidino)phosphoniumchlorid
Tri(bis(methyl-tetraethoxyethylen)amino)-(pyrrolidino)phosphoniumchlorid
Morpholino-tris(pyrrolidino)phosphoniumchlorid
Dimorpholino-bis(pyrrolidino)phosphoniumchlorid
Trimorpholino-(pyrrolidino)phosphoniumchlorid

Bis(2-methoxyethyl)amino-tris(piperidino)phosphoniumchlorid
Di(bis(2-methoxyethyl)amino)-bis(piperidino)phosphoniumchlorid
Tri(bis(2-methoxyethyl)amino)-(piperidino)phosphoniumchlorid
Bis(methyl-diethoxyethylen)amino-tris(piperidino)phosphoniumchlorid
Di(bis(methyl-diethoxyethylen)amino)-bis(piperidino)phosphoniumchlorid
Tri(bis(methyl-diethoxyethylen)amino)-(piperidino)phosphoniumchlorid
Bis(methyl-tetraethoxyethylen)amino-tris(piperidino)phosphoniumchlorid
Di(bis(methyl-tetraethoxyethylen)amino)-bis(piperidino)phosphoniumchlorid
Tri(bis(methyl-tetraethoxyethylen)amino)-(piperidino)phosphoniumchlorid
Morpholino-tris(piperidino)phosphoniumchlorid
Dimorpholino-bis(piperidino)phosphoniumchlorid

Bis(2-methoxyethyl)amino-tris(diethylamino)phosphoniumchlorid
Di(bis(2-methoxyethyl)amino)-bis(diethylamino)phosphoniumchlorid
Tri(bis(2-methoxyethyl)amino)-(diethylamino)phosphoniumchlorid
Bis(methyl-tetraethoxyethylen)amino-tris(diethylamino)phosphoniumchlorid
Di(bis(methyl-tetraethoxyethylen)amino)-bis(diethylamino)phosphoniumchlorid
Tri(bis(methyl-tetraethoxyethylen)amino)-(diethylamino)phosphoniumchlorid
Morpholino-tris(diethylamino)phosphoniumchlorid
Dimorpholino-bis(diethylamino)phosphoniumchlorid
Trimorpholino-(diethylamino)phosphoniumchlorid

Bis(2-methoxyethyl)amino-tris(dimethylamino)phosphoniumchlorid
Di(bis(2-methoxyethyl)amino)-bis(dimethylamino)phosphoniumchlorid
Tri(bis(2-methoxyethyl)amino)-(dimethylamino)phosphoniumchlorid
Bis(methyl-bisethoxyethylen)amino-tris(dimethylamino)phosphoniumchlorid
Di(bis(methyl-bisethoxyethylen)amino)-bis(dimethylamino)phosphoniumchlorid
Tri(bis(methyl-bisethoxyethylen)amino)-(dimethylamino)phosphoniumchlorid
Bis(methyl-tetraethoxyethylen)amino-tris(dimethylamino)phosphoniumchlorid

Di(bis(methyl-tetraethoxyethylen)amino)-bis(dimethylamino)phosphoniumchlorid
Tri(bis(methyl-tetraethoxyethylen)amino)-(dimethylamino)phosphoniumchlorid
Tris(dimethylamino)-morpholino-phosphoniumchlorid
Bis(dimethylamino)-dimorpholino-phosphoniumchlorid
Dimethylamino-trimorpholino-phosphoniumchlorid

Bis(2-methoxyethyl)amino-tris(butyl-ethylamino)phosphoniumchlorid
Di(bis(2-methoxyethyl)amino)-bis(butyl-ethylamino)phosphoniumchlorid
Tri(bis(2-methoxyethyl)amino)-(butyl-ethylamino)phosphoniumchlorid
Bis(methyl-tetraethoxyethylen)amino-tris(butyl-ethylamino)phosphoniumchlorid
Di(bis(methyl-tetraethoxyethylen)amino)-bis(butyl-ethylamino)phosphoniumchlorid
Tri(bis(methyl-tetraethoxyethylen)amino)-(butyl-ethylamino)phosphoniumchlorid
Tris(butyl-ethylamino)morpholino-phosphoniumchlorid
Bis(butyl-ethylamino)-dimorpholino-phosphoniumchlorid
(Butyl-ethylamino)-trimorpholino-phosphoniumchlorid
N-Methylpiperazino-tris(pyrrolidino)phosphoniumchlorid
Bis(N-methylpiperazino)-bis(pyrrolidino)phosphoniumchlorid
Tris(N-methylpiperazino)-(pyrrolidino)phosphoniumchlorid
N-Methylpiperazino-tris(diethylamino)phosphoniumchlorid
Bis(N-methylpiperazino)-bis(diethylamino)phosphoniumchlorid
Tris(N-methylpiperazino)-(diethylamino)phosphoniumchlorid
N-Methylpiperazino-tris(dimethylamino)phosphoniumchlorid
Bis(N-methylpiperazino)-bis(dimethylamino)phosphoniumchlorid
Tris(N-methylpiperazino)-(dimethylamino)phosphoniumchlorid
N-Methylpiperazino-tris(butyl-ethylamino)phosphoniumchlorid
Bis(N-methylpiperazino)-bis(butyl-ethylamino)phosphoniumchlorid
Tris(N-methylpiperazino)-(butyl-ethylamino)phosphoniumchlorid

Anstelle der aufgeführten Aminophosphoniumchloride können z.B. auch die entsprechenden Bromide, Iodide, Fluoride, Sulfate, Hydrogensulfate, Acetate oder Phthalate eingesetzt werden.

Die vorliegende Erfindung betrifft femer Stoffgemische enthaltend wenigstens eine Verbindung der allgemeinen Formel 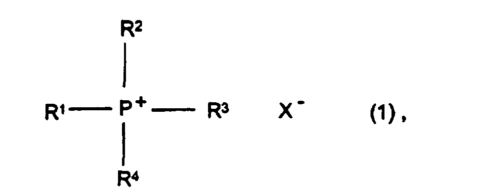 worin R¹, R², R³, R⁴ und X⁻ die vorstehende Bedeutung haben und wenigstens eine Verbindung ausgewählt aus der Gruppe von quartären Ammoniumverbindungen der Formel 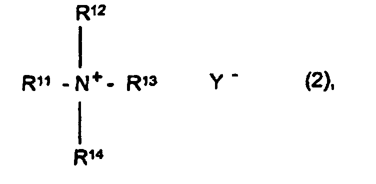 quartären Ammoniumsalzen oder Phosphoniumsalzen der Formel

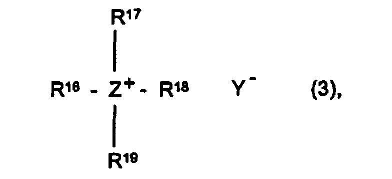

Polyethern der Formel R²⁰-(O-CₓH₂ₓ)ₛ-OR²¹ (4) und Kronenethem,
worin in Formel (2) R¹¹, R¹² und R¹³ gleich oder verschieden sind und einen linearen oder verzweigten Rest der Formel -(CₚH₂ₚO)ᵣR¹⁵ bedeuten, worin R¹⁵ für Wasserstoff oder einen linearen oder verzweigten Alkylrest mit 1 bis 16 Kohlenstoffatomen steht, p eine ganze Zahl von 1 bis 10 und r eine ganze Zahl von 1 bis 15 bedeuten;
oder einen linearen oder verzweigten Alkylrest mit 1 bis 30 Kohlenstoffatomen; oder einen unsubstituierten Phenyl- oder Naphthylrest, oder einen substituierten Phenyl- oder Naphthylrest, wobei die Substituenten die Bedeutung Halogen, C₁-C₄-Alkyl, C₁-C₄-Alkoxy, Nitro oder Cyano haben;
R¹⁴ einen linearen oder verzweigten Rest der Formel -(CₚH₂ₚO)ᵣR¹⁵ bedeutet; und
Y⁻ ein anorganisches Anion ist;
und in Formel (3)
R¹⁶, R¹⁷, R¹⁸ und R¹⁹ gleich oder verschieden sind und einen linearen oder verzweigten Alkylrest mit 1 bis 22 Kohlenstoffatomen bedeuten; oder einen unsubstituierten oder substituierten Arylrest oder einen C₁-C₄-Alkylarylrest, wobei Aryl die Bedeutung Phenyl oder Naphthyl hat und die besagten Substituenten Halogen,
C₁-C₄-Alkyl, C₁-C₄-Alkoxy, Nitro oder Cyano bedeuten; Z die Bedeutung N oder P hat und Y⁻ ein anorganisches Anion ist;
und in Formel (4)
R²⁰ und R²¹ gleich oder verschieden sind und einen linearen oder verzweigten Alkylrest mit 1 bis 16 Kohlenstoffatomen bedeuten;
x eine ganze Zahl von 2 bis 6 und
s eine ganze Zahl von 1 bis 60 ist;
oder einer der Reste R²⁰ und R²¹ Wasserstoff und der andere der Reste einen linearen oder verzweigten Alkylrest mit 1 bis 16 Kohlenstoffatomen bedeutet, x eine ganze Zahl von 2 bis 6 und s eine ganze Zahl von 2 bis 50 ist, oder die Reste R²⁰ und R²¹ Wasserstoff bedeuten, x eine ganze Zahl von 2 bis 6 und s eine ganze Zahl von 3 bis 5 ist.

Die vorliegende Erfindung betrifft insbesondere Stoffgemische, die wenigstens eine Verbindung der Formel (1) und wenigstens eine Verbindung ausgewählt aus der Gruppe von quartären Ammoniumverbindungen der Formel (2), quartären Ammoniumsalzen und Phosphoniumsalzen der Formel (3), Polyethern der Formel (4) und Kronenethem enthalten, worin in Formel (2) R¹¹, R¹² und R¹³ gleich oder verschieden sind und einen linearen oder verzweigten Rest der Formel -(CₚH₂ₚO)ᵣR¹⁵ bedeuten, worin R¹⁵ für Wasserstoff oder einen linearen oder verzweigten Alkylrest mit 1 bis 8 Kohlenstoffatomen steht, p eine ganze Zahl von 1 bis 5 und r eine ganze Zahl von 2 bis 10 bedeuten; oder einen linearen oder verzweigten Alkylrest mit 1 bis 18 Kohlenstoffatomen; oder einen unsubstituierten Phenyl- oder Naphthylrest; R¹⁴ einen linearen oder verzweigten Rest der Formel -(CₚH₂ₚO)ᵣR¹⁵ bedeutet, worin R¹⁵ für Wasserstoff oder einen linearen oder verzweigten Alkylrest mit 1 bis 8 Kohlenstoffatomen steht, p eine ganze Zahl von 1 bis 5 und r eine ganze Zahl von 2 bis 10 bedeuten; und X⁻ Fluorid, Chlorid, Bromid, SO₄²⁻/2 oder Hydrogensulfat ist.

Besonders interessant sind Stoffgemische, die wenigstens eine Verbindung der Formel (1) und wenigstens eine Verbindung aus der Gruppe der quartären Ammoniumverbindungen der Formel (2) enthalten.

Zu erwähnen sind auch Stoffgemische der vorstehenden Art, die wenigstens ein Ammoniumsalz oder Phosphoniumsalz der Formel (3) enthalten, worin in Formel (3) R¹⁶, R¹⁷, R¹⁸ und R¹⁹ gleich oder verschieden sind und einen linearen oder verzweigten Alkylrest mit 1 bis 16 Kohlenstoffatomen bedeuten; oder einen unsubstituierten oder substituierten Arylrest oder einen C₁-C₄-Alkylarylrest, wobei Aryl die Bedeutung Phenyl oder Naphthyl hat und die besagten Substituenten Halogen, C₁-C₄-Alkyl, C₁-C₄-Alkoxy, Nitro oder Cyano bedeuten.

Stoffgemische der vorstehenden Art, die wenigstens einen Polyether der Formel (4) oder Kronenether enthalten, worin in Formel (4) R²⁰ und R²¹ gleich oder verschieden sind und Wasserstoff oder einen linearen oder verzweigten Alkylrest mit 1 bis 8 Kohlenstoffatomen, x eine ganze Zahl von 2 bis 3 und s eine ganze Zahl von 4 bis 50 bedeuten, sind ebenfalls von Interesse.

Die Stoffgemische enthalten üblicherweise 5 bis 95 Gew.-%, insbesondere 20 bis 80 Gew.-%, bevorzugt 25 bis 75 Gew.-% wenigstens einer Verbindung der allgemeinen Formel (1). Die verbleibenden 95 bis 5 Gew.-%, insbesondere 80 bis 20 Gew.-%, bevorzugt 75 bis 25 Gew.-% der Stoffgemische entfallen auf den Rest, nämlich auf wenigstens eine Verbindung ausgewählt aus der Gruppe der quartären Ammoniumverbindungen der Formel (2), der quartären Ammonium- oder Phosphoniumsalze der Formel (3), Polyethem der Formel (4) und Kronenethem, insbesondere auf wenigstens eine Verbindung aus der Gruppe der quartären Ammoniumverbindungen der Formel (2).

Die vorliegende Erfindung betrifft femer ein Verfahren zur Herstellung von Verbindungen der Formel

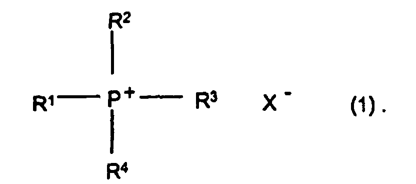

Es ist dadurch gekennzeichnet, daß man ein Phosphorpentahalogenid in Gegenwart eines inerten Lösungsmittels bei -70 bis 140°C, insbesondere -30 bis 120°C, bevorzugt -15 bis 60°C, mit 1 bis 6 Mol, insbesondere 1 bis 2,5 Mol 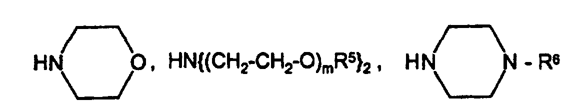 oder HN{(CH₂-CH₂-N(R⁷))ₙR⁸}, je auszutauschendem Äquivalent Halogen und anschließend das Reaktionsprodukt mit 1 bis 10 Mol, insbesondere 1 bis 3 Mol 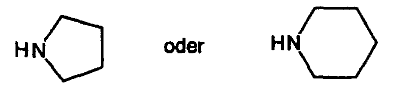 oder HNR⁹R¹⁰ je noch auszutauschendem Äquivalent Halogen umsetzt. Diese Umsetzung des Reaktionsproduktes erfolgt bei -15 bis 140°C, insbesondere 0 bis 130°C, bevorzugt 20 bis 130°C.

Man setzt als Phosphorpentahalogenid PCl₅ oder PBr₅, insbesondere PCl₅ ein.

Man setzt als inertes Lösungsmittel einen aliphatischen, cycloaliphatischen oder aromatischen Kohlenwasserstoff oder einen einfach oder mehrfach chlorierten aliphatischen, cycloaliphatischen oder aromatischen Kohlenwasserstoff ein.

Gut geeignet als inertes Lösungsmittel sind z.B. Hexan, Cyclohexan, Methylcyclohexan, Toluol, Ethylbenzol, Mesitylen, o-Xylol, m-Xylol, p-Xylol, technische Gemische isomerer Xylole, Methylenchlorid, Tetrachlorethan, Chlorbenzol, Dichlorbenzol oder Chlortoluol. Es lassen sich auch Gemische von Lösungsmitteln verwenden.

Man suspendiert oder löst das Phosphorpentahalogenid in dem inerten Lösungsmittel und führt anschließend die Umsetzung unter Zugabe der vorstehend genannten Stickstoff enthaltenden Verbindungen durch.

Man erhält auf diese Weise Verbindungen der Formel (1), worin X⁻ für ein Halogenid steht. Falls gewünscht kann das Halogenid durch Umsalzen gegen ein anderes der vorstehend genannten Anionen ausgetauscht werden.

Die Erfindung betrifft femer die Verwendung der Verbindungen der Formel (1) als Katalysator und Cokatalysator für Phasentransferreaktionen, nucleophile Substitutionen und Halogen-Fluor-Austauschreaktionen.

Die Erfindung betrifft weiterhin die Verwendung der Stoffgemische enthaltend wenigstens eine Verbindung der Formel (1) und wenigstens eine Verbindung ausgewählt aus der Gruppe von quartären Ammoniumverbindungen der Formel (2), quartären Ammoniumsalzen oder Phosphoniumsalzen der Formel (3), Polyethern der Formel (4) und Kronenethem als Katalysator für Phasentransferreaktionen, nucleophile Substitutionen und Halogen-Fluor-Austauschreaktionen.

Die nachfolgenden Beispiele beschreiben die vorliegende Erfindung näher, ohne sie zu beschränken.

### Experimenteller Teil

### Herstellung der Aminophosphoniumverbindungen (Aminophosphoniumsalze)

### Beispiel 1: Bis(2-methoxyethyl)amino-tris(pyrrolidino)phosphoniumchlorid

104,12 g (0,5 mol) Phosphorpentachlorid werden in 400 g Chlorbenzol eingetragen und auf -20 bis -10°C gekühlt. Bei dieser Temperatur werden 66,89 g (0,5 mol) Bis-(2-methoxyethyl)amin so zugetropft, daß die Innentemperatur 0°C nicht überschreitet. Nach der Zugabe läßt man langsam auf 30°C erwärmen und erhitzt dann für eine Stunde auf 90-100°C.
Dann wird wieder auf 10°C gekühlt und es werden 214,55 g (3,0 mol) Pyrrolidin so zugetropft, daß die Innentemperatur 15°C nicht überschreitet. Nach der Zugabe erhitzt man für zwei Stunden zum Rückfluß.
Nach Abkühlen auf 0°C wird vorsichtig mit 779,94 g (3,9 mol) 20%iger Natronlauge alkalisch gestellt. Die wäßrige Phase wird abgetrennt und verworfen. Anschliessend wird überschüssiges Amin von der organischen Phase abdestilliert. Nach dem Ende der Destillation wird mit HCl auf pH=6-7 angesäuert, filtriert und das Chlorbenzol am Rotationsverdampfer abgezogen.
Elementaranalyse: C=55,2%, H=8,9%, N=12,9%, P=7,0%, Cl=8,2%

### Beispiel 2: Bis(bis(2-methoxyethyl)amino)-bis(pyrrolidino)phosphoniumbromid

104,12 g (0,5 mol) Phosphorpentachlorid werden in 400 g Chlorbenzol eingetragen und auf -20 bis -10°C gekühlt. Bei dieser Temperatur werden 133,78 g (1 mol) Bis-(2-methoxyethyl)amin so zugetropft, daß die Innentemperatur 0°C nicht überschreitet. Nach der Zugabe erhitzt man für eine Stunde auf 100°C.
Dann wird wieder auf 10°C gekühlt und es werden 214,55 g (3,0 mol) Pyrrolidin so zugetropft, daß die Innentemperatur 15°C nicht überschreitet. Nach der Zugabe erhitzt man für zwei Stunden zum Rückfluß.
Nach Abkühlen auf 0°C wird vorsichtig mit 779,94 g (3,9 mol) 20%iger Natronlauge (entspricht 155,99 g NaOH) alkalisch gestellt. Die wäßrige Phase wird abgetrennt und verworfen. Nach Zugabe von 71,93 g (0,6 mol) Kaliumbromid wird von der organischen Phase überschüssiges freies Amin abdestilliert. Nach dem Ende der Destillation wird mit HBr auf pH=6-7 angesäuert, filtriert und das Chlorbenzol am Rotationsverdampfer abgezogen.
Elementaranalyse: C=46,9%, H=8,8%, N=10,6%, P=5,9%, Br=15,2%, Cl=0,14%

### Beispiel 3: (n-Butyl-ethylamino)-tris-(pyrrolidino)phosphoniumbromid

Zu 20,8 g (0,1 mol) PCl₅ in 100 ml Chlorbenzol tropft man in 1 Stunde 42,7 g (0,6 mol) Pyrrolidin so zu, daß die Innentemperatur 15°C nicht übersteigt. Nach der Zugabe wird 1 Stunde bei 50°C nachgerührt, dann erfolgt bei 25°C Reaktionstemperatur die Zugabe von 16 g (0,22 mol) n-Butylamin zu.
Nach 1 Stunde unter Rückfluß wird erneut auf 25°C abgekühlt, mit 200 ml Eiswasser hydrolysiert und die wässrige Phase abgetrennt.
Nach Abdestillieren des Aminüberschusses wird 19,9 g (0,5 mol) NaOH in 20 ml Wasser eintragen. Bei 50°C erfolgt tropfenweise die Zugabe von 12,0 g (0,11 mol) Ethylbromid. Nach 3 Stunden Nachrühren bei 70°C wird auf 25°C abgekühlt und mit 200 ml Wasser verdünnt. Die organische Phase wird abgetrennt und mit 60%iger Bromwasserstoffsäure auf pH=6-7 angesäuert. Nach Abdestillieren aller flüchtigen Bestandteile erhält man 33,6 g (n-Butyl-ethylamino)-tris-(pyrrolidino)-phosphoniumbromid als leicht bräunliches Öl.

### Beispiel 4: Bis(methyl-tetraethoxyethylen)amino-tris(pyrrolidino)-phosphoniumchlorid

Bis(methyl-tetraethoxyethylen)amino-tris(pyrrolidino)phosphoniumchlorid wird analog Beispiel 1 aus 104,12 g (0,5 mol) Phosphorpentachlorid, 198,75 g (0,5 mol) Bis-(methyl-tetraethoxyethylen)amin (=HN{(CH₂-CH₂-O)₄CH₃}₂) und 214,55 g (3,0 mol) Pyrrolidin hergestellt.
Elementaranalyse: C=53,6%, H=9,6%, N=8,1%, P=4,3%, Cl=5,6%

### Beispiel 5: Bis(bis(methyl-tetraethoxyethylen)amino)-bis(pyrrolidino)-phosphoniumchlorid

Bis(bis(methyl-tetraethoxyethylen)amino)-bis(pyrrolidino)phosphoniumchlorid wird analog Beispiel 2 aus 104,12 g (0,5 mol) Phosphorpentachlorid, 397,51 g (1 mol) Bis-(methyl-tetraethoxyethylen)amin (=HN{(CH₂-CH₂-O)₄CH₃}₂) und 214,55 g (3,0 mol) Pyrrolidin hergestellt.
Elementaranalyse: C=44,7%, H=8,3%, N=6,5%, P=3,7%, Cl=4,5%

### Beispiel 6: Tris(2-methoxyethyl)amino-(diethylamino)phosphoniumbromid

104,12 g (0,5 mol) Phosphorpentachlorid werden in 400 g Chlorbenzol eingetragen und auf -20 bis -10°C gekühlt. Bei einer Reaktionstemperatur von < 5°C tropft man 401,34 g (3 mol) Bis-(2-methoxyethyl)amin zu. Nach der Zugabe läßt man langsam auf 30°C erwärmen und erhitzt dann für eine Stunde auf 100°C.
Bei 20°C werden 36g (2,1 mol) Ammoniak eingeleitet. Nach Hydrolyse mit 850 g 20%iger wässriger Natronlauge wird das Chlorbenzol und der Überschuß an Bis-(2-methoxyethyl)amin von der organische Phase abdestilliert (120°C, 10 mbar). Der ölige Rückstand wird in 300 ml Chlorbenzol aufgenommen und mit 340 g 50%iger wässriger Natronlauge versetzt. Bei 50°C tropft man 119 g (1,1 mol) Ethylbromid zu, zur Vervollständigung der Reaktion wird noch 3 Stunden bei 70°C nachgerührt. Dann wird bei 20°C 350 ml Wasser zugesetzt, nach Phasentrennung wird die organische Phase mit 24 g 36%iger Bromwasserstoffsäure neutralisiert. Nach Abdestillieren aller flüchtigen Bestandteile erhält man 33,6 g Tris(2-methoxyethyl)amino-(diethylamino)phosphoniumbromid als bräunliches Öl.
Elementaranalyse: C=36,1%, H=7,9%, N=7,5%, P=4,15%, Br=9,8%, Cl=0,5%.

### Beispiel 7: N-Methylpiperazino-tris(pyrrolidino)phosphoniumchlorid

41,65 g (0,2 mol) Phosphorpentachlorid werden in 200 g Toluol eingetragen und auf -20°C gekühlt. Dann werden 20,03 g (0,2 mol) N-Methylpiperazin so zugetropft, daß die Innentemperatur 5°C nicht überschreitet. Nach der Zugabe wird für eine Stunde auf 100°C erhitzt.
Bei 10°C tropft man 56,9 g (0,8 mol) Pyrrolidin zu. Nach der Zugabe läßt man langsam auf 20°C erwärmen. Nach weiteren 30 min wird für zwei Stunden zum Rückfluß erhitzt.
Nach Hydrolyse mit 20%iger wässriger Natronlauge wird das Toluol und der Aminüberschuß von der organischen Phase abdestilliert (120°C, 10 mbar). Man erhält 69,6 g N-Methylpiperazino-tris(pyrrolidino)phosphoniumchlorid als bräunliches Öl.
Elementaranalyse: C=54,3%, H=9,6%, N=18,5%, P=8,15%, Cl=9,7%

### Beispiel 8: Dioctylamino-tris(pyrrolidino)phosphoniumchlorid

Dioctylamino-tris(pyrrolidino)phosphoniumchlorid wird analog Beispiel 1 aus 41,65 g (0,2 mol) Phosphorpentachlorid, 48,3 g (0,2 mol) Dioctylamin und 99,6 g (1,4 mol) Pyrrolidin hergestellt.
Elementaranalyse: C=65,3%, H=11,6%, N=10,5%, P=5,9%, Cl=7,0%

### Beispiel 9: Morpholino-tris(pyrrolidino)phosphoniumchlorid

Morpholino-tris(pyrrolidino)phosphoniumchlorid wird analog Beispiel 1 aus 104,12 g (0,5 mol) Phosphorpentachlorid, 43,6 g (0,5 mol) Morpholin und 214,6 g (3,0 mol) Pyrrolidin hergestellt.
Elementaranalyse: C=52,8%, H=9,0%, N=15,3%, P=8,4%, Cl=10,1%
Das entsprechende Bromid wird durch Salzmetathese analog Beispiel 2 erhalten.

### Beispiel 10: Bis(morpholino)-bis(pyrrolidino)phosphoniumchlorid

Bis(morpholino)-bis(pyrrolidino)phosphoniumchlorid wird analog Beispiel 2 aus 104,12 g (0,5 mol) Phosphorpentachlorid, 87,12 g (1 mol) Morpholin und 214,55 g (3,0 mol) Pyrrolidin hergestellt.
Elementaranalyse: C=50,6%, H=8,7%, N=14,6%, P=8,0%, Cl=9,5%
Das entsprechende Bromid wird durch Salzmetathese analog Beispiel 2 erhalten.

### Beispiel 11: Bis(2-methoxyethyl)amino-tris(piperidino)phosphoniumchlorid

Bis(2-methoxyethyl)amino-tris(piperidino)phosphoniumchlorid wird analog Beispiel 2 aus 104,12 g (0,5 mol) Phosphorpentachlorid, 66,9 g (0,5 mol) Bis-(2-methoxyethyl)amin und 255,4 g (3,0 mol) Piperidin hergestellt.

### Beispiel 12: Bis(bis(2-methoxyethyl)amino)-bis(piperidino)phosphoniumchlorid

Bis(bis(2-methoxyethyl)amino)-bis(piperidino)phosphoniumchlorid wurde analog Beispiel 1 aus 104,12 g (0,5 mol) Phosphorpentachlorid, 133,2 g (1 mol) Bis-(2-methoxyethyl)amin und 212,9 g (2,5 mol) Piperidin hergestellt.

### Vergleichsbeispiel A: Tetrakis(diethylamino)phosphoniumbromid (Vergleichssubstanz)

Zu 52,1 g (0,25 mol) PCl₅ in 220 ml Chlorbenzol tropft man in 1 Stunde 109,7 g (1,5 mol) Diethylamin so zu, daß die Innentemperatur 5°C nicht übersteigt. Nach der Zugabe wird 1 Stunde bei 30°C nachgerührt, dann wird bei T=15°C 30 g Ammoniak eingeleitet.
Nach 1 Stunde werden 340 g 20%iger Natronlauge zugegeben und die wässrige Phase abgetrennt. Von der organische Phase wird das überschüssige Diethylamin abdestilliert. Dann werden 170 g 50%iger Natronlauge und 60 g (0,55 mol) Ethylbromid versetzt und für 4 Sunden auf 50°C erwärmt. Nach Phasentrennung wird die organische Phase mit 60%iger Bromwasserstoffsäure auf pH=6-7 angesäuert. Nach Abdestillieren aller flüchtigen Bestandteile erhält man 83,9 g Tetrakis(diethylamino)phosphoniumbromid als leicht bräunliches Öl.

### Vergleichsbeispiel B: Tris(dibutylamino)-(diethylamino)phosphoniumbromid (Vergleichssubstanz)

Tris(dibutylamino)-(diethylamino)phosphoniumbromid wird analog Vergleichsbeispiel A aus 41,65 g (0,2 mol) Phosphorpentachlorid, 77,50 g (0,6 mol) Dibutylamin, einem Überschuß Ammoniak und 47,9 g (0,44 mol) Ethylbromid hergestellt.
Elementaranalyse: C=53,2%, H=10,9%, N=12,3%, P=6,75%, Br=16,3%, Cl=0,7%

### Austestung der katalytischen Aktivität:

### Herstellung von 2,6-Difluorbenzaldehyd (DFBAL) aus 2-Chlor-6-fluorbenzaldehyd (CFBAL) mittels Chlor-Fluor-Austauschreaktion

### Vergleichsbeispiel 1 A und 1 B

Zu 158,6 g (1 mol) 2-Chlor-6-fluorbenzaldehyd (CFBAL) werden nacheinander bei 60°C 4,4 g (0,01 mol) Tetrakis-(diethylamino)phosphoniumbromid (Vergleichsbeispiel 1 A) oder 5,7 g (0,01 mol) Tris(dibutylamino)-(diethylamino)-phosphoniumbromid (Vergleichsbeispiel 1 B) und jeweils 17,6 g (0,03 mol) Methyltris-(methyltetraethoxy)-ammoniumchlorid [{CH₃-(O-C₂H₄)₄}₃NCH₃]Cl, 58,3 g (1 mol) Kaliumfluorid und 10 ml Chlorbenzol zugegeben. Durch Abdestillieren des Chlorbenzols bei vermindertem Druck wird die Reaktionsmischung azeotrop getrocknet. Nach 20 Stunden bei 170°C wird die Bildung 2,6-Difluorbenzaldehyd (DFBAL) und der Umsatz der Reaktion gaschromatographisch bestimmt.

### Beispiele

In den folgenden in Tabelle 1 durch ein * gekennzeichneten Beispielen wird anstelle von Tetrakis(diethylamino)phosphoniumbromid eine molar entsprechende Menge des in Tabelle 1 jeweils angegebenen Aminophosphoniumsalzes (jeweils 0,01 mol) und 0,03 mol Methyl-tris(methyltetraethoxy)ammoniumchlorid eingesetzt. Sonst wird analog Vergleichsbeispiel 1A verfahren.
Die Zahlenangaben in Tabelle 1 entsprechen GC-Flächenprozent. Die in Tabelle 1 ebenfalls angegebene Differenz zu 100% (Rest) ist ein Maß für Nebenreaktionen und Zersetzung.

**Tabelle 1**

| Ausbeuten 2,6-Difluorbenzaldehyd (DFBAL) aus der Halex-Reaktion von 2-Chlor-6-fluorbenzaldehyd (CFBAL) in GC-Flächen-% | | | |
|---|---|---|---|
| Aminophosphoniumsalz | DFBAL | CFBAL | Rest |
| ohne Katalysatoren | 16,2 | 72,1 | 11,7 |
| [(Et₂N)₄P]Br | 56,1 | 24,7 | 19,2 |
| [(Bu₂N)₃(Et₂N)P]Br | 63,0 | 20,1 | 16,9 |
| [(Me(OCH₂CH₂)₂N)(Pyrrolidino)₃P]Br * | 68,9 | 15,0 | 16,1 |
| [(Me(OCH₂CH₂)₂N)(Pyrrolidino)₃P]Cl * | 67,7 | 17,8 | 14,5 |
| [(Me(OCH₂CH₂)₂N)₂(Pyrrolidino)₂P]Br * | 70,2 | 12,3 | 17,5 |
| [{(Et)(Bu)N}(Pyrrolidino)₃P]Br * | 64,4 | 22,6 | 13,0 |
| [{(Me(O-C₂H₄)₄)₂N}(Pyrrolidino)₃P]Br * | 71,5 | 18,4 | 10,1 |
| [{(Me(O-C₂H₄)₄)₂N}₂(Pyrrolidino)₂P]Br * | 70,8 | 17,2 | 12,0 |
| [(N-Me(Piperazino)(Pyrrolidino)₃P]Cl * | 68,2 | 18,2 | 13,6 |
| [(Morpholino)(Pyrrolidino)₃P]Br * | 68,5 | 18,1 | 13,4 |
| [(Morpholino)(Pyrrolidino)₃P]Cl * | 68,2 | 17,1 | 14,7 |
| [(Morpholino)₂(Pyrrolidino)₂P]Br * | 66,3 | 19,7 | 14,0 |
| [(Morpholino)₂(Pyrrolidino)₂P]Cl * | 65,8 | 20,1 | 14,1 |
| [(Me(OCH₂CH₂)₂N)(Piperidino)₃P]Cl * | 71,2 | 16,8 | 12,0 |
| [(Me(OCH₂CH₂)₂N)₂(Piperidino)₂P]Cl * | 70,4 | 18,2 | 11,4 |

| | | | |
|---|---|---|---|
| * Beispiele | | | |

### Austestung der katalytischen Aktivität:

### Herstellung von 2,6-Difluorbenzaldehyd (DFBAL) und 2-Chlor-6-fluorbenzaldehyd (CFBAL) aus 2,6-Dichlorbenzaldehyd (DCBAL) mittels Chlor-Fluor-Austauschreaktion.

### Vergleichsbeispiel 2 A und 2 B

Zu 175,0 g (1 mol) 2,6-Dichlorbenzaldehyd (DCBAL) werden nacheinander bei 60°C 8,8 g (0,02 mol) Tetrakis-(diethylamino)phosphoniumbromid (Vergleichsbeispiel 2 A) oder 11,35 (0,02 mol) Tris(dibutylamino)(diethylamino)phosphoniumbromid (Vergleichsbeispiel 2 B) und jeweils 35,2 g (0,06 mol) Methyltris(methyltetraethoxy)ammoniumchlorid, 116,2 g (2 mol) Kaliumfluorid und 10 ml Chlorbenzol zugegeben. Durch Abdestillieren des Chlorbenzols bei vermindertem Druck wird die Reaktionsmischung azeotrop getrocknet. Nach 20 Stunden bei 165°C wird die Bildung von 2-Chlor-6-fluorbenzaldehyd (CFBAL) und 2,6-Difluorbenzaldehyd (DFBAL) und der Umsatz der Reaktion gaschromatographisch bestimmt.

### Beispiele

In den folgenden in Tabelle 2 durch ein * gekennzeichneten Beispielen wird anstelle von Tetrakis-(diethylamino)-phosphoniumbromid eine molar entsprechende Menge des jeweils angegebenen Aminophosphoniumsalzes (jeweils 0,02 mol) und 0,06 mol Methyl-tris(methyltetraethoxy)ammoniumchlorid eingesetzt. Sonst wird analog Vergleichsbeispiel 2 A verfahren.
Die Zahlenangaben in Tabelle 2 entsprechen GC-Flächenprozent. Die in Tabelle 2 ebenfalls angegebene Differenz zu 100% (Rest) ist ein Maß für Nebenreaktionen und Zersetzung.

**Tabelle 2**

| Ausbeuten 2,6-Difluorbenzaldehyd (DFBAL) und 2-Chlor-6-fluorbenzaldehyd (CFBAL) aus der Halex-Reaktion von 2,6-Dichlorbenzaldehyd (DCBAL) in GC-Flächen-% | | | | |
|---|---|---|---|---|
| Aminophosphoniumsalz | DFBAL | CFBAL | DCBAL | Rest |
| ohne Katalysator | 1,2 | 11,4 | 71,5 | 15,9 |
| [(Et₂N)₄P]Br | 40,2 | 26,9 | 11,2 | 21,7 |
| [(Bu₂N)₃(Et₂N)P]Br | 41,1 | 27,5 | 10,4 | 21,0 |
| [(Me(OCH₂CH₂)₂N)(Pyrrolidino)₃P]Br * | 51,1 | 36,1 | 1,8 | 11,0 |
| [(Me(OCH₂CH₂)₂N)(Pyrrolidino)₃P]Cl * | 50,7 | 37,8 | 2,1 | 9,4 |
| [(Me(OCH₂CH₂)₂N)₂(Pyrrolidino)₂P]Br * | 50,6 | 37,3 | 1,4 | 10,7 |
| [{(Et)(Bu)N}(Pyrrolidino)₃P]Br * | 43,6 | 31,2 | 8,6 | 16,6 |
| [(Octyl₂N)(Pyrrolidino)₃P]Br * | 41,6 | 33,5 | 8,1 | 16,8 |
| [{(Me(O-C₂H₄)₄)₂N}(Pyrrolidino)₃P]Br * | 53,8 | 31,4 | 3,9 | 10,9 |
| [{(Me(O-C₂H₄)₄)₂N}₂(Pyrrolidino)₂P]Br * | 50,4 | 36,8 | 2,1 | 10,7 |
| [(N-Me(Piperazino)(Pyrrolidino)₃P]Cl * | 46,9 | 38,2 | 4,4 | 10,5 |
| [(Morpholino)(Pyrrolidino)₃P]Br * | 46,9 | 32,3 | 5,1 | 15,7 |
| [(Morpholino)(Pyrrolidino)₃P]Cl * | 47,2 | 34,8 | 5,4 | 12,6 |
| [(Morpholino)₂(Pyrrolidino)₂P]Br * | 47,8 | 35,6 | 5,2 | 11,4 |
| [(Morpholino)₂(Pyrrolidino)₂P]Cl * | 49,6 | 31,9 | 4,8 | 13,7 |
| [(Me(OCH₂CH₂)₂N)(Piperidino)₃P]Cl * | 54,2 | 34,4 | 1,7 | 9,7 |
| [(Me(OCH₂CH₂)₂N)₂(Piperidino)₂P]Cl * | 53,1 | 35,5 | 1,5 | 9,9 |

| | | | | |
|---|---|---|---|---|
| * Beispiele | | | | |

In den Tabellen 1 und 2 stehen Me für Methyl, Et für Ethyl und Bu für Butyl, Me(O-C₂H₄)₄ für Methyl-tetraethoxyethylen
Pyrrolidino für 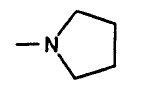 Morpholino für 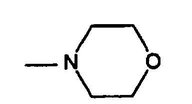 N-Me(Piperazino) für 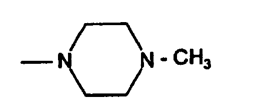 und Piperidino für

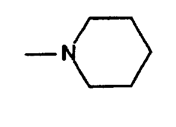

## Patentansprüche

1. Verbindungen der allgemeinen Formel 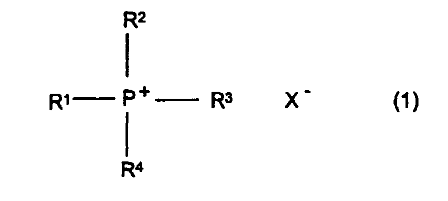 worin einer, zwei oder drei der Reste R¹, R², R³ und R⁴ für 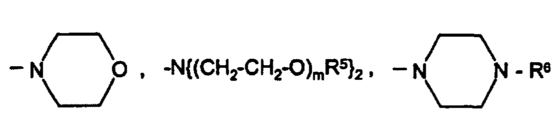 oder -N{(CH₂-CH₂-N(R⁷))ₙR⁸}₂ stehen, wobei m und n eine ganze Zahl von 1 bis 10 bedeuten, R⁵, R⁶, R⁷ und R⁸ unabhängig voneinander gleich oder verschieden sind und einen geradkettigen oder verzweigten Alkylrest mit 1 bis 10 C-Atomen bedeuten, und der oder die verbleibenden Reste R¹ bis R⁴ für 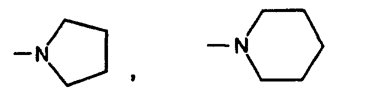 oder -NR⁹R¹⁰, wobei R⁹ und R¹⁰ gleich oder verschieden sind und einen geradkettigen oder verzweigten Alkylrest mit 1 bis 10 C-Atomen bedeuten, stehen und X⁻ ein anorganisches oder organisches Anion oder ein Äquivalent eines mehrwertigen anorganischen oder organischen Anions ist.

2. Verbindungen nach Anspruch 1, **dadurch gekennzeichnet, daß** ein oder zwei der Reste R¹, R², R³ und R⁴ für 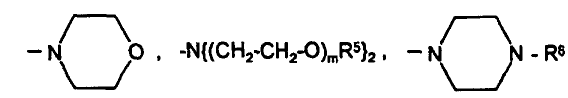 oder -N{(CH₂-CH₂-N(R⁷))ₙR⁸}₂ und die verbleibenden Reste R¹ bis R⁴ für 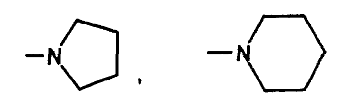 oder -NR⁹R¹⁰ stehen.

3. Verbindungen nach Anspruch 1 oder 2, **dadurch gekennzeichnet, daß** m und n eine ganze Zahl von 1 bis 6 bedeuten und R⁵, R⁶, R⁷, R⁸, R⁹ und R¹⁰ gleich oder verschieden sind und für einen geradkettigen oder verzweigten Alkylrest mit 1 bis 4 C-Atomen stehen.

4. Verbindungen nach einem oder mehreren der Ansprüche 1 bis 3, **dadurch gekennzeichnet, daß** X⁻ für F⁻, Cl⁻, Br⁻, J⁻, ClO₄⁻, BF₄⁻, PF₆⁻, NO₃⁻, HSO₄⁻, ½ SO₄²⁻, H₂PO₄⁻, ½ HPO₄²⁻, 1/3 PO₄³⁻, R'-COO⁻, wobei R' einen Alkylrest mit 1 bis 9 C-Atomen, einen Phenylrest, Benzylrest oder Naphthylrest bedeutet, R''-SO₃ ⁻, wobei R" einen Alkylrest mit 1 bis 18 C-Atomen, einen Phenylrest, Tolylrest oder Naphthylrest bedeutet, HCO₃⁻, ½ CO₃²⁻ oder für ½ C₆H₄(COO⁻)₂ steht.

5. Stoffgemische enthaltend wenigstens eine Verbindung der allgemeinen Formel 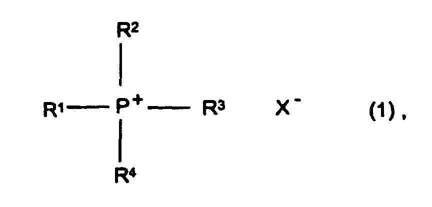 worin R¹, R², R³, R⁴ und X⁻ die vorstehende Bedeutung haben und wenigstens eine Verbindung ausgewählt aus der Gruppe von quartären Ammoniumverbindungen der Formel 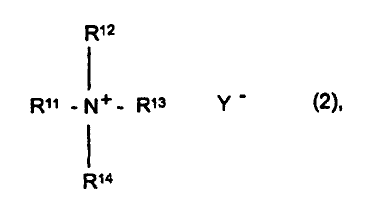 quartären Ammoniumsalzen oder Phosphoniumsalzen der Formel 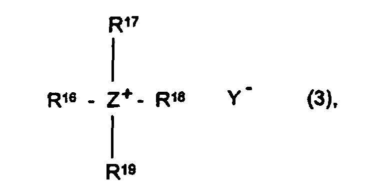 Polyethem der Formel R²⁰-(O-CₓH₂ₓ)ₛ-OR²¹ (4) und Kronenethern,
worin in Formel (2) R¹¹, R¹² und R¹³ gleich oder verschieden sind und einen linearen oder verzweigten Rest der Formel -(CₚH₂ₚO)ᵣR¹⁵ bedeuten, worin R¹⁵ für Wasserstoff oder einen linearen oder verzweigten Alkylrest mit 1 bis 16 Kohlenstoffatomen steht, p eine ganze Zahl von 1 bis 10 und r eine ganze Zahl von 1 bis 15 bedeuten;
oder einen linearen oder verzweigten Alkylrest mit 1 bis 30 Kohlenstoffatomen;
oder einen unsubstituierten Phenyl- oder Naphthylrest, oder einen substituierten Phenyl- oder Naphthylrest, wobei die Substituenten die Bedeutung Halogen, C₁-C₄-Alkyl, C₁-C₄-Alkoxy, Nitro oder Cyano haben;
R¹⁴ einen linearen oder verzweigten Rest der Formel -(CₚH₂ₚO)ᵣR¹⁵ bedeutet; und
Y⁻ ein anorganisches Anion ist;
und in Formel (3)
R¹⁶, R¹⁷, R¹⁸ und R¹⁹ gleich oder verschieden sind und einen linearen oder verzweigten Alkylrest mit 1 bis 22 Kohlenstoffatomen bedeuten; oder einen unsubstituierten oder substituierten Arylrest oder einen C₁-C₄-Alkylarylrest, wobei Aryl die Bedeutung Phenyl oder Naphthyl hat und die besagten Substituenten Halogen, C₁-C₄-Alkyl, C₁-C₄-Alkoxy, Nitro oder Cyano bedeuten;Z die Bedeutung N oder P hat und Y⁻ ein anorganisches Anion ist;
und in Formel (4)
R²⁰ und R²¹ gleich oder verschieden sind und einen linearen oder verzweigten Alkylrest mit 1 bis 16 Kohlenstoffatomen bedeuten;
x eine ganze Zahl von 2 bis 6 und
s eine ganze Zahl von 1 bis 60 ist;
oder einer der Reste R²⁰ und R²¹ Wasserstoff und der andere der Reste einen linearen oder verzweigten Alkylrest mit 1 bis 16 Kohlenstoffatomen bedeutet, x eine ganze Zahl von 2 bis 6 und s eine ganze Zahl von 2 bis 50 ist, oder die Reste R²⁰ und R²¹ Wasserstoff bedeuten, x eine ganze Zahl von 2 bis 6 und s eine ganze Zahl von 3 bis 5 ist.

6. Stoffgemische nach Anspruch 5, **dadurch gekennzeichnet, daß** sie wenigstens eine Verbindung der Formel (1) und wenigstens eine Verbindung ausgewählt aus der Gruppe von quartären Ammoniumverbindungen der Formel (2), quartären Ammoniumsalzen und Phosphoniumsalzen der Formel (3), Polyethem der Formel (4) und Kronenethem enthalten, worin in Formel (2) R¹¹, R¹² und R¹³ gleich oder verschieden sind und einen linearen oder verzweigten Rest der Formel -(CₚH₂ₚO)ᵣR¹⁵ bedeuten, worin R¹⁵ für Wasserstoff oder einen linearen oder verzweigten Alkylrest mit 1 bis 8 Kohlenstoffatomen steht, p eine ganze Zahl von 1 bis 5 und r eine ganze Zahl von 2 bis 10 bedeuten; oder einen linearen oder verzweigten Alkylrest mit 1 bis 18 Kohlenstoffatomen; oder einen unsubstituierten Phenyl- oder Naphthylrest;
R¹⁴ einen linearen oder verzweigten Rest der Formel -(CₚH₂ₚO)ᵣR¹⁵ bedeutet, worin
R¹⁵ für Wasserstoff oder einen linearen oder verzweigten Alkylrest mit 1 bis 8 Kohlenstoffatomen steht, p eine ganze Zahl von 1 bis 5 und r eine ganze Zahl von 2 bis 10 bedeuten; und
X⁻ Fluorid, Chlorid, Bromid, SO₄²⁻/2 oder Hydrogensulfat ist.

7. Stoffgemische nach Anspruch 5 oder 6, **dadurch gekennzeichnet, daß** sie wenigstens eine Verbindung der Formel (1) und wenigstens eine Verbindung aus der Gruppe der quartären Ammoniumverbindungen der Formel (2) enthalten.

8. Verfahren zur Herstellung von Verbindungen der Formel 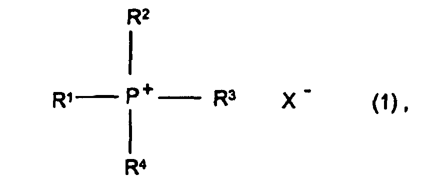 **dadurch gekennzeichnet, daß** man ein Phosphorpentahalogenid in Gegenwart eines inerten Lösungsmittels bei -70 bis 140°C mit 1 bis 6 Mol 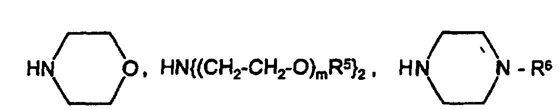 oder HN{(CH₂-CH₂-N(R⁷))ₙR⁸}, je auszutauschendem Äquivalent Halogen und anschließend das Reaktionsprodukt mit 1 bis 10 Mol 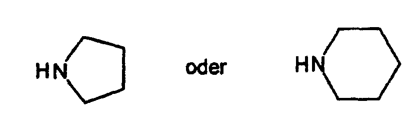 oder HNR⁹R¹⁰ je noch auszutauschendem Äquivalent Halogen umsetzt.

9. Verfahren nach Anspruch 8, **dadurch gekennzeichnet, daß** man als Phosphorpentahalogenid PCl₅ oder PBr₅ einsetzt.

10. Verfahren nach Anspruch 8 oder 9, **dadurch gekennzeichnet, daß** man a inertes Lösungsmittel einen aliphatischen, cycloaliphatischen oder aromatische Kohlenwasserstoff oder einen einfach oder mehrfach chlorierten aliphatischen, cycloaliphatischen oder aromatischen Kohlenwasserstoff einsetzt.

11. Verwendung der Verbindungen der Formel (1) als Katalysator und Cokatalysator für Phasentransferreaktionen, nucleophile Substitutionen und Halogen-Fluor-Austauschreaktionen.

12. Verwendung der Stoffgemische enthaltend wenigstens eine Verbindung der Formel (1) und wenigstens eine Verbindung ausgewählt aus der Gruppe von quartären Ammoniumverbindungen der Formel (2), quartären Ammoniumsalzen oder Phosphoniumsalzen der Formel (3), Polyethem der Formel (4) und Kronenethern als Katalysator für Phasentransferreaktionen, nucleophile Substitutionen und Halogen-Fluor-Austauschreaktionen.

## Claims

1. A compound of the formula 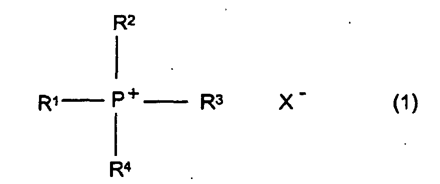 in which one, two or three of the radicals R¹, R², R³ and R⁴ are 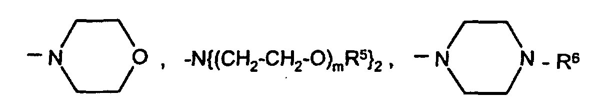 or -N{(CH₂-CH₂-N(R⁷))ₙR⁸}₂ where m and n are an integer from 1 to 10, R⁵, R⁶, R⁷ and R⁸ are, independently of one another, identical or different and are a straight-chain or branched alkyl radical having 1 to 10 carbon atoms, and the remaining radical(s) R¹ to R⁴ are 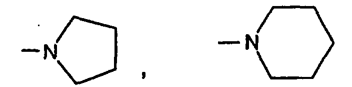 or -NR⁹R¹⁰, where R⁹ and R¹⁰ are identical or different and are a straight-chain or branched alkyl radical having 1 to 10 carbon atoms,
and X⁻ is an inorganic or organic anion or an equivalent of a multiply charged inorganic or organic anion.

2. A compound as claimed in claim 1, wherein one or two of the radicals R¹, R², R³ and R⁴ are 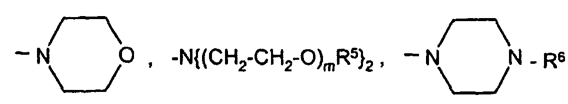 or -N{(CH₂-CH₂-N(R⁷))ₙR⁸}₂ and the remaining radicals R¹ to R⁴ are 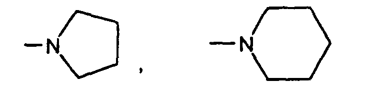 or -NR⁹R¹⁰.

3. A compound as claimed in claim 1 or 2, wherein m and n are an integer from 1 to 6, and R⁵, R⁶, R⁷, R⁸, R⁹ and R¹⁰ are identical or different and are a straight-chain or branched alkyl radical having 1 to 4 carbon atoms.

4. A compound as claimed in one or more of claims 1 to 3, wherein X⁻ is F⁻, Cl⁻, Br⁻, I⁻, ClO₄ ⁻, BF₄ ⁻, PF₆ ⁻, NO₃⁻, HSO₄⁻, ½ SO₄²⁻, H₂PO₄⁻, ½ HPO₄²⁻, 1/3 PO₄³⁻, R'-COO⁻ , where R' is an alkyl radical having 1 to 9 carbon atoms,
a phenyl radical, benzyl radical or naphthyl radical, R"-SO₃⁻, where R" is an alkyl radical having 1 to 18 carbon atoms, a phenyl radical, tolyl radical or naphthyl radical, HCO₃⁻, ½ CO₃²⁻ or ½ C₆H₄(COO⁻)₂.

5. A mixture of substances comprising at least one compound of the formula 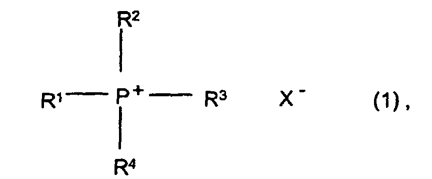 in which R¹, R², R³, R⁴ and X⁻ have the above meaning, and at least one compound selected from the group of quaternary ammonium compounds of the formula 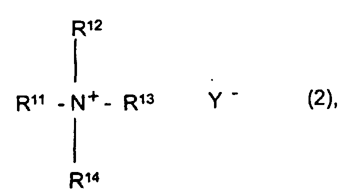 quaternary ammonium salts or phosphonium salts of the formula 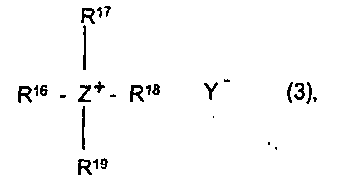 polyethers of the formula R²⁰-(O-CₓH₂ₓ)ₛ-OR²¹ (4) and crown ethers,
in which in formula (2) R¹¹, R¹² and R¹³ are identical or different and are a linear or branched radical of the formula -(CₚH₂ₚO)ᵣR¹⁵ in which R¹⁵ is hydrogen or a linear or branched alkyl radical having 1 to 16 carbon atoms, p is an integer from 1 to 10 and r is an integer from 1 to 15;
or a linear or branched alkyl radical having 1 to 30 carbon atoms;
or an unsubstituted phenyl or naphthyl radical, or a substituted phenyl or naphthyl radical, where the substituents have the meaning of halogen, C₁-C₄-alkyl, C₁-C₄-alkoxy, nitro or cyano;
R¹⁴ is a linear or branched radical of the formula -(CₚH₂ₚO)ᵣR¹⁵ and
Y⁻ is an inorganic anion;
and in formula (3)
R¹⁶, R¹⁷, R¹⁸ and R¹⁹ are identical or different and are a linear or branched alkyl radical having 1 to 22 carbon atoms; or an unsubstituted or substituted aryl radical or a C₁-C₄-alkylaryl radical, where aryl has the meaning of phenyl or naphthyl, and said substituents are halogen,
C₁-C₄-alkyl, C₁-C₄-alkoxy, nitro or cyano; Z has the meaning of N or P, and Y⁻ is an inorganic anion;
and in formula (4)
R²⁰ and R²¹ are identical or different and are a linear or branched alkyl radical having 1 to 16 carbon atoms;
x is an integer from 2 to 6 and
s is an integer from 1 to 60;
or one of the radicals R²⁰ and R²¹ is hydrogen and the other one of the radicals is a linear or branched alkyl radical having 1 to 16 carbon atoms,
x is an integer from 2 to 6 and s is an integer from 2 to 50, or the radicals R²⁰ and R²¹ are hydrogen, x is an integer from 2 to 6 and s is an integer from 3 to 5.

6. A mixture of substances as claimed in claim 5, which comprises at least one compound of the formula (1) and at least one compound selected from the group of quaternary ammonium compounds of the formula (2), quaternary ammonium salts and phosphonium salts of the formula (3), polyethers of the formula (4) and crown ethers, in which in formula (2) R¹¹, R¹² and R¹³ are identical or different and are a linear or branched radical of the formula -(CₚH₂ₚO)ᵣR¹⁵ in which R¹⁵ is hydrogen or a linear or branched alkyl radical having 1 to 8 carbon atoms, p is an integer from 1 to 5 and r is a number from 2 to 10; or a linear or branched alkyl radical having 1 to 18 carbon atoms; or an unsubstituted phenyl or naphthyl radical; R¹⁴ is a linear or branched radical of the formula -(CₚH₂ₚO)ᵣR¹⁵, in which R¹⁵ is hydrogen or a linear or branched alkyl radical having 1 to 8 carbon atoms, p is an integer from 1 to 5 and r is an integer from 2 to 10; and X⁻ is fluoride, chloride, bromide, SO₄²⁻/2 or hydrogen sulfate.

7. A mixture of substances as claimed in claim 5 or 6, which comprises at least one compound of the formula (1) and at least one compound from the group of quaternary ammonium compounds of the formula (2).

8. A process for preparing compounds of the formula 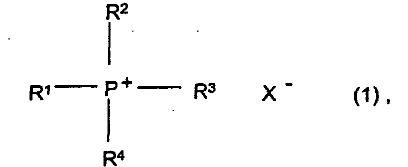 which comprises reacting a phosphorus pentahalide in the presence of an inert solvent at from -70 to 140°C with from 1 to 6 mol of 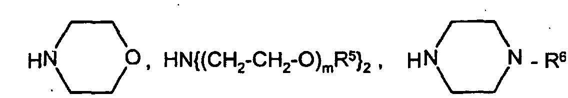 or HN{(CH₂-CH₂-N(R⁷))ₙR⁸}, per halogen equivalent to be exchanged, and subsequently reacting the reaction product with from 1 to 10 mol of 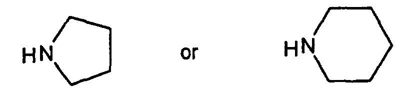 or HNR⁹R¹⁰ per halogen equivalent still to be exchanged.

9. The process as claimed in claim 8, wherein PCl₅ or PBr₅ is employed as phosphorus pentahalide.

10. The process as claimed in claim 8 or 9, wherein an aliphatic, cycloaliphatic or aromatic hydrocarbon or a mono- or polychlorinated aliphatic, cycloaliphatic or aromatic hydrocarbon is employed as inert solvent.

11. The use of a compound of the formula (1) as catalyst and cocatalyst for phase-transfer reactions, nucleophilic substitutions and halogen-fluorine exchange reactions.

12. The use of a mixture of substances comprising at least one compound of the formula (1) and at least one compound selected from the group of quaternary ammonium compounds of the formula (2), quaternary ammonium salts or phosphonium salts of the formula (3), polyethers of the formula (4) and crown ethers as catalyst for phase-transfer reactions, nucleophilic substitutions and halogen-fluorine exchange reactions.

## Revendications

1. Composés de formule générale 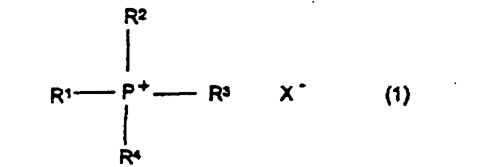 où un, deux ou trois des restes R¹, R², R³ et R⁴ représentent 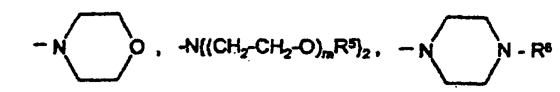 ou -N{(CH₂-CH₂-N(R⁷))ₙR⁸}₂ m et n sont un entier de 1 à 10, R⁵, R⁶, R⁷ et R⁸ indépendamment l'un de l'autre identiques ou différents représentent un reste alkyle linéaire ou ramifié présentant 1 à 10 atomes de carbone, et le ou les autres restes R¹ à R⁴ représentent 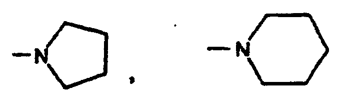 ou -NR⁹R¹⁰, R⁹ et R¹⁰ sont identiques ou différents et représentent un reste alkyle linéaire ou ramifié présentant 1 à 10 atomes de carbone,
et X⁻ représente un anion inorganique ou organique ou un équivalent d'un anion multivalent inorganique ou organique.

2. Composés selon la revendication 1, **caractérisés en ce que** un ou deux des restes R¹, R², R³ et R⁴ représentent 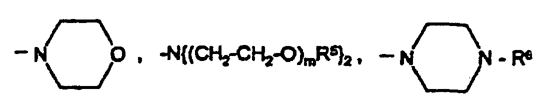 ou : -N{(CH₂-CH₂-N(R⁷))ₙR⁸}₂ les autres restes R¹ à R⁴ représentent 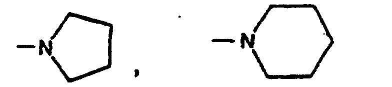 ou -NR⁹R¹⁰.

3. Composés selon la revendication 1 ou 2, **caractérisés en ce que** m et n sont des entiers de 1 à 6 et R⁵, R⁶, R⁷, R⁸, R⁹ et R¹⁰ identiques ou différents représentent un reste alkyle linéaire ou ramifié présentant 1 à 4 atomes de carbone.

4. Composés selon une ou plusieurs des revendications 1 à 3, **caractérisés en ce que** X⁻ représente F⁻, Cl⁻, Br⁻, I⁻, ClO₄⁻, BF₄⁻, PF₆⁻, NO₃⁻, HSO₄⁻, ½SO₄²⁻, H₂PO₄⁻, ½HPO₄²⁻, ¹/₃PO₄³⁻, R' -COO⁻, où R' représente un reste alkyle présentant 1 à 9 atomes de carbone, un reste phényle, un reste benzyle ou un reste naphtyle, R"-SO₃⁻, R" représente un reste alkyle présentant 1 à 18 atomes de carbone, un reste phényle, un reste tolyle ou un reste naphtyle, HCO₃⁻, ½CO₃²⁻ ou ½C₆H₄(COO⁻)₂·

5. Mélanges renfermant au moins un composé de formule générale 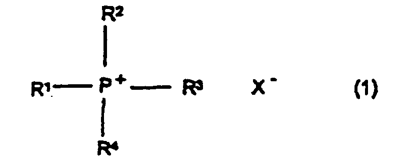 où R¹, R², R³, R⁴ et X⁻ possèdent la signification donnée ci-dessus et au moins un composé est pris dans le groupe des composés d'ammonium quaternaires de formule 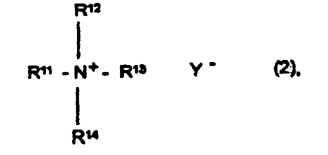 des sels d'ammonium quaternaires ou de phosphonium de formule 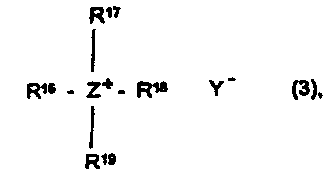 des polyéthers de formule R²⁰(O-CₓH₂ₓ)ₛ-OR²¹ (4) et des éthers couronnes où à la formule (2) R¹¹, R¹² et R¹³ identiques ou différents représentent un reste linéaire ou ramifié de formule -(CₚH₂ₚO)ᵣR¹⁵, où R¹⁵ représente un atome d'hydrogène ou un reste alkyle linéaire ou ramifié présentant 1 à 16 atomes de carbone, p est un entier de 1 à 10 et r est un entier de 1 à 15 ;
ou un reste alkyle linéaire ou ramifié présentant 1 à 30 atomes de carbone ;
ou un reste phényle ou naphtyle non substitué ou un reste phényle ou naphtyle substitué, les substituants sont un halogène, un alkyle en C₁-C₄, un alkoxy en C₁-C₄, un nitro ou cyano ;
R¹⁴ représente un reste linéaire ou ramifié de formule -(CₚH₂ₚO)ᵣR¹⁵ ; et
Y⁻ est un anion inorganique ;
et à la formule (3)
R¹⁶, R¹⁷, R¹⁸ et R¹⁹ sont identiques ou différents et représentent un reste alkyle linéaire ou ramifié présentant 1 à 22 atomes de carbone ; ou un reste aryle non substitué ou substitué ou un reste (alkyl en C₁-C₄)aryle, le reste aryle est un groupe phényle ou naphtyle et lesdits substituants sont un halogène, un alkyle en C₁-C₄, un alkoxy en C₁-C₄, un nitro ou cyano ;
Z représente N ou P et
Y⁻ est un anion inorganique ;
et à la formule (4)
R²⁰ et R²¹ sont identiques ou différents et représentent un reste alkyle linéaire ou ramifié présentant 1 à 16 atomes de carbone ;
x est un entier de 2 à 6 et
s est un entier de 1 à 60 ;
ou un des restes R²⁰ et R²¹ représente un atome d'hydrogène et l'autre reste un reste alkyle linéaire
ou ramifié de 1 à 16 atomes de carbone ;
x est un entier de 2 à 6 et s un entier de 2 à 50, ou les restes
R²⁰ et R²¹ représentent un atome d'hydrogène, x est un entier de 2 à 6 et s est un entier de 3 à 5.

6. Mélanges de matières selon la revendication 5, **caractérisés en ce qu'**ils renferment au moins un composé de formule (1) et au moins un composé pris dans le groupe comprenant des composés d'ammonium quaternaires de formule (2), des sels d'ammonium quaternaires et de phosphonium de formule (3), des polyéthers de formule (4) et des éthers couronnes, où à la formule (2) R¹¹, R¹² et R¹³ sont identiques ou différents et représentent un reste linaire ou ramifié de formule -(CₚH₂ₚO)ᵣR¹⁵, où R¹⁵ représente un atome d'hydrogène ou un reste alkyle linaire ou ramifié présentant 1 à 8 atomes de carbone, p est un entier de 1 à 5 et r est un entier de 2 à 10 ; ou un reste alkyle linéaire ou ramifié présentant 1 à 18 atomes de carbone ; ou un reste phényle ou naphtyle non substitué ;
R¹⁴ représente un reste linaire ou ramifié de formule -(CₚH₂ₚO)ᵣR¹⁵, où R¹⁵ représente un atome d'hydrogène ou un reste alkyle linaire ou ramifié présentant 1 à 8 atomes de carbone, p est un entier de 1 à 5 et r est un entier de 2 à 10 ; et
X⁻ représente un fluorure, chlorure, bromure, SO₄²⁻/2 ou bisulfate.

7. Mélanges de matières selon la revendication 5 ou 6, **caractérisés en ce qu'**ils renferment au moins un composé de formule (1) et au moins un composé pris dans le groupe des composés d'ammonium quaternaires de formule (2).

8. Procédé pour la préparation de composés de formule 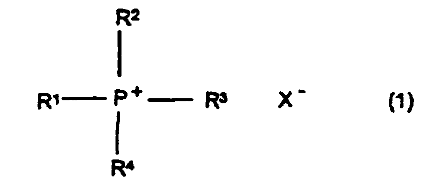 **caractérisé en ce qu'**on fait réagir un pentahalogénure de phosphore en présence d'un solvant inerte, à une température de -70 à 140°C, sur 1 à 6 moles de 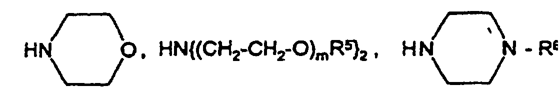 ou HN{(CH₂-CH₂-N(R⁷))ₙR⁸}₂ par équivalent d'halogène à échanger et ensuite on fait réagir le produit réactionnel sur 1 à 10 moles de 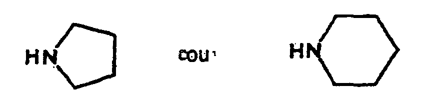 ou HNR⁹R¹⁰ par équivalent d'halogène à échanger encore.

9. Procédé selon la revendication 8, **caractérisé en ce qu'**on utilise comme pentahalogénure de phosphore PCl₅ ou PBr₅.

10. Procédé selon la revendication 8 ou 9, **caractérisé en ce qu'**on utilise comme solvant inerte un hydrocarbure aliphatique, cycloaliphatique ou aromatique ou un hydrocarbure aliphatique, cycloaliphatique ou aromatique chloré une ou plusieurs fois.

11. Utilisation des composés de formule (1) en tant que catalyseur et co-catalyseur de réactions de transfert de phase, de substitutions nucléophiles et de réactions d'échange halogène-fluor.

12. Utilisation de mélanges de matières renfermant au moins un composé de formule (1) et au moins un composé pris dans le groupe des composés d'ammonium quaternaire de formule (2), de sels d'ammonium quaternaires ou des sels de phosphonium de formule (3), de polyéthers de formule (4) et d'éthers couronnes en tant que catalyseur de réactions de transfert de phase, de substitutions nucléophiles et de réactions d'échange halogène-fluor.
